# EUROPEAN PATENT SPECIFICATION

(11) **EP 0 497 723 B1**
(45) Date of publication and mention of the grant of the patent: **27.11.1996**
(21) Application number: 92500010.1
(22) Date of filing: 27.01.1992
(51) Int. Cl.: A61M 39/00

(54) **Disinfecting connection for catheters and the like**
Desinfektierende Kupplung für Katheter und dergleichen
Raccord désinfectant pour cathéters et similaires

(30) Priority: 30.01.1991 ES 9100239
(43) Date of publication of application: 05.08.1992
(73) Proprietor: Segura Badia, Marcelo, ES-08009 Barcelona (ES); INSTITUT MUNICIPAL D'ASSISTENCIA SANITARIA, ES-08009 Barcelona (ES)
(72) Inventor: Segura Badia, Marcelo, ES-08009 Barcelona (ES)
(74) Representative: Klunker . Schmitt-Nilson . Hirsch

(56) References cited:
- EP-A- 0 256 640
- EP-A- 0 298 257
- FR-A- 2 506 162
- US-A- 4 354 490

## Description

This invention describes a disinfecting connection for catheters and the like which has substantial advantages over what has been previously known in this technical field. The connection which is the subject of this invention will be applied to conductors for catheters and other similar means by which the passage of fluid products to the human body is to be carried out. As examples of such products, different drug solutions, blood serum and others may be mentioned.

In such conductors for fluids to be incorporated to the human body, it is necessary to provide means for the disinfection of the connection element, which usually consists of a needle.

EP-A-298 257 discloses an embodiment of an invention of the same Applicant in which a solution is given to the problem of the complete and integral disinfection of the needle to be connected to catheters. However, in this previous embodiment there was the problem of the possible physical contact of the hand or other external bodies with the limiting end closure of the disinfecting element, whose protection was not complete. Also, the centering of the connecting needle with the disinfecting element was not completely safe because the needle was not properly guided during its insertion. This could lead to a faulty location of the needle, making a new insertion necessary.

Additionally, in the previously known embodiment, when the need came to replace the disinfecting unit, the catheter which formed part of the unit was to be replaced as well, which made a new surgical intervention for the insertion of the catheter on the patient necessary. This is especially important in cases of patients requiring the application of the catheter for very long periods of time which in some cases may be years or may extend to the whole life of the patient.

This invention brings improvements to what is known nowadays in this field, providing a complete connection for pipe conductors, the connection may be exchanged as a whole and has means for holding a disinfecting unit, which unit may be readily replaced by a new one and also the necessary means for the connection of the conductor to convey the solutions or perfusion liquids to the human body.

Essentially, the connection of this invention consists of two parts, each to be incorporated respectively to one of the ends of two conductors in a catheter arrangement and which may be attached in a removable or fixed form to a body carrying a disinfecting means which will have therefore the possibility of being exchanged without it being necessary to change the catheter. Such disinfecting means carrying body, or disinfecting element, consists of a small cylinder having end closures, preferably made out of an elastic material, which may be easily penetrated by a needle incoporated to one of the ends of the above mentioned conductors. Said internal cylinder is filled with the disinfecting liquid. When coupling the needle to the central body carrying the disinfecting elements the needle will penetrate the elastic closures of the disinfecting element, reaching a position to allow the passage of the liquids which have to be perfused to the human body, whilst maintaining the necessary sterilized conditions.

For the renewal of the disinfecting means when it is desired, it is sufficient to disconnect the central body carrying the disinfecting element and replace the disinfecting element with a new one or replace the whole central body. Alternatively, the renewal of the disinfecting element may be carried out together with the whole connection which is attached to the conductors.

An alternative embodiment of the present invention provides the coupling of the parts enclosing the disinfecting means by non-detachable welding, preventing eventual serious accidents in case that in the dismantling of the conductor carrying the needle, such dismantling might be accidentally carried out at the level of the attachment of both parts enclosing the disinfecting unit whose contents could be spilled out or could enhance the entrance of air due to the dislodging.

Therefore, the present invention comprises a disinfecting connection means according to claim 1. Further embodiments of the disinfecting connecting means are in the dependant claims.

For better understanding, some explanatory drawings are enclosed, which have to be understood as non limitative examples of disinfecting connections which incorporate the features of this invention.

Figure 1 shows a longitudinal section of a connection according to the present invention.

Figures 2, 3, 4, 5 and 6 are cross-sections in the planes indicated in figure 1.

Figure 7 is a section of the connection part receiving the needle.

Figure 8 is an upper view corresponding to figure 7.

Figures 9 and 10 correspond respectively to a longitudinal section and to an upper view of the cap carrying the connection needle.

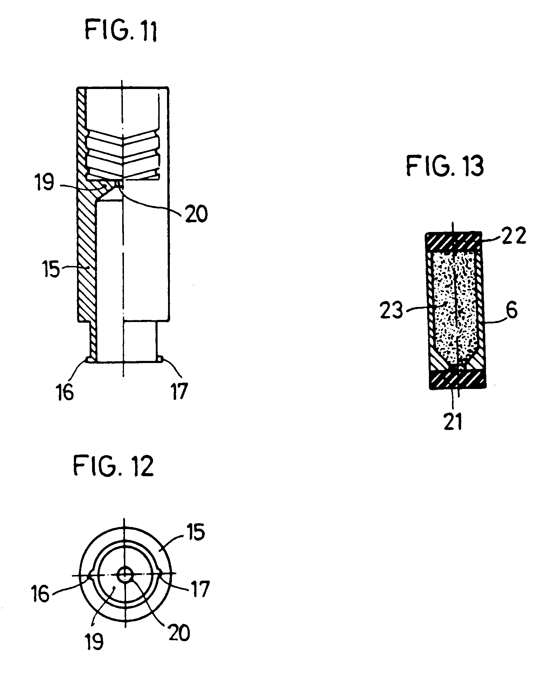

Figures 11 and 12 correspond to a longitudinal section and to an upper view, respectively, of one of the parts forming the body which carries the disinfecting element.

Figure 13 corresponds to a longitudinal section of the disinfecting element.

Figures 14 and 15 show respectively a longitudinal section and an upper view of the second part forming the body carrying the disinfecting element.

Figures 16 and 17 correspond respectively to a longitudinal section and to an upper view of the terminal portion of the catheter tube.

Figures 18 and 19 correspond respectively to a longitudinal section and to an upper view of the body of the connection carrying the disinfecting unit.

Figure 20 corresponds to a cross section of an alternative embodiment of this invention.

According to the drawings, the disinfecting connection of this invention consists of a first and second terminal, 1 and 2 respectively, forming part of or to be attached to the pieces of connector 3 and 4 and to be connected to the catheter tube body 5 carrying the eventually exchangeable disinfecting element 6.

The arrangement is such as to allow the ready assembly and disassembly of the connection, obtaining at the same time the disinfecting effect on the needle and the easy renewal of the disinfecting unit which are the objectives of the invention.

To that end, the second terminal 2 is attached by one of its ends to an expansion 7 of tube 4. The other end of the second terminal has a cuplike part 8 having internal attachment grooves and having an internal tubular extension 9 aimed at receiving the base 10 of needle 11, which base will be pressure fitted to the outside of said tubular extension 9 of the second terminal 2, thereby abutting the end of said terminal 2.

In this way, the tube 4 and the second terminal 2 together with the needle, constitute one of the parts to be attached to the central body 5. Whenever a new perfusion cycle has to be started, the second terminal 2 will be disassembled and assembled anew on the central body 5.

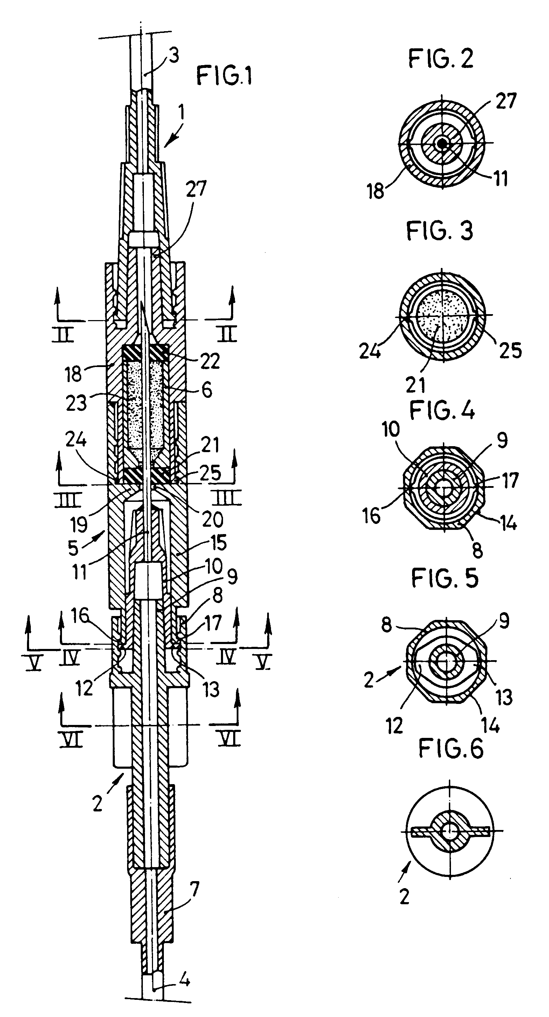

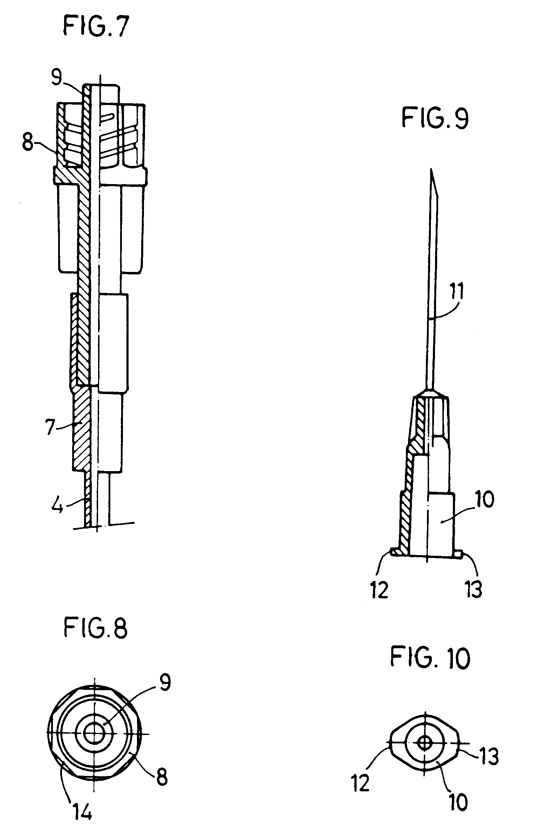

For the quick coupling of the needle base 10 with the grooving of the cuplike part 8, said base 10 has two opposed diametral protrusions 12 and 13 in its base, which mate with the grooving of the cup-like part 8 as may be seen in figures 1 and 8.

The cuplike part 8 has on its outside surface flat faces 14 to ease its attachment and disattachment by hand.

The central body 5 which houses the disinfecting element 6 is constituted by parts 15 and 18 which, in this embodiment, are pressure fitted or engaged by means of screwing.

The part 15 has internally a partition 19 with a hole 20 to allow the guidance and passage of the needle, and in its lower part, it has two protrusions 16 and 17, aimed at engaging the internal threading of the second terminal 2.

The disinfecting element 6, which is interchangeable, contains the disinfecting liquid and has elastic closures 21 and 22 on its ends, which will be penetrated by the needle.

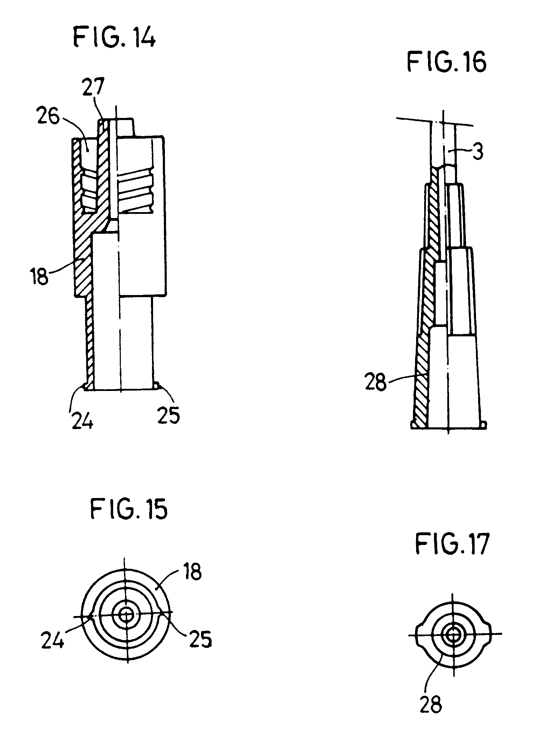

Figure 14 shows the part 18 of the central body 5 having, in its lower rim, two protrusions 24 and 25, and in the other end, a housing 26 with internal threading. From the part 18, a central extension 27 protrudes upwards, being aimed at receiving the first terminal 1, which has an internally tapering housing 28 mating with the external conical form of the extension 27.

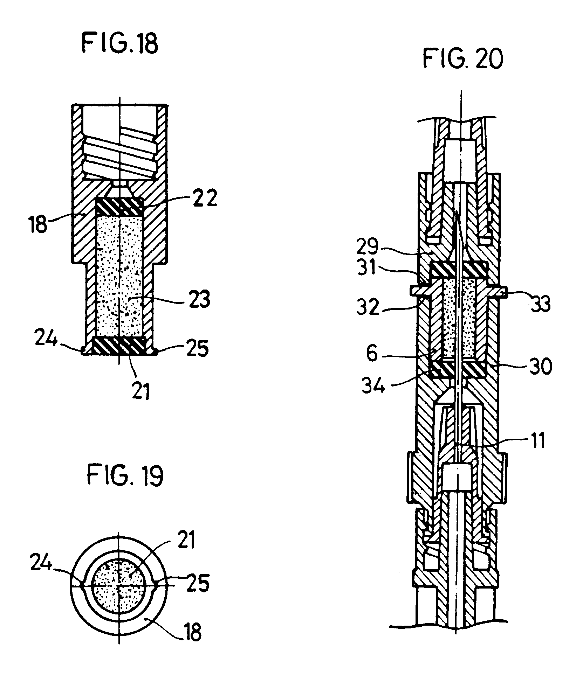

The embodiment of figure 20 shows the arrangement of the disinfecting element 6 between an upper part and a lower part respectively 29 and 30 of the connection. The attachment of such parts will be fixed, by means of welding or similar means, in the abutment faces 31 corresponding to upper part 29 and 32 corresponding to lower part 30. The disinfecting element 6 carrying the disinfecting product has a protruding ring 33 which exceeds slightly the external diameter of upper and lower parts 29 and 30 providing the abutment faces. The attachment of both parts 29 and 30 could also be carried out directly

It is possible in this way to establish an efficient protection against physical contact on the end closure 34 of the disinfecting unit. At the same time, the accidental disassembly of the end closure for the disinfecting unit will be prevented.

By the above described means, it will be easy to carry out the quick engagement and disengagement of the two conductors enabling the disinfecting unit to be easily and aseptically interchanged.

As it will be understood, many modifications could be carried out in the above described embodiment of this invention, without departing from the scope of the following claims:

## Claims

1. Disinfecting connection for catheters and the like which comprises a chamber containing a disinfecting product **characterized in that** said chamber is a multiple-use disinfecting element (6) contained inside a corresponding space of a longitudinal, central body (5), said body comprising a first part (18) and a second part (15) engageable together to form said space, said central body (5) receiving in one of its ends a first terminal (1) which is coupled to a first catheter tube (3) and receiving its other end a second terminal (2), which is coupled to a second tube (4) and which second terminal is carrying a needle (11) destinated for traversing said chamber-like multiple-use disinfecting element (6).

2. Disinfecting connection for catheters and the like according to claim 1, **characterized in that** the second terminal (2) carrying the needle (11) is pressure fitted by one of its ends to an expansion (7) of the corresponding second tube (4), while in the other end it has a cuplike part (8) with internal threading for receiving the corresponding part of the central body (5) carrying the disinfecting element (6), the second terminal (2) having as well an axial expansion (9) protruding from the cuplike part (8) and allowing for pressure engagement of the base of the needle (11).

3. Disinfecting connection for catheters and the like according to claim 1, **characterized in that** the second part (15) of the central body (5) mates with the internal threading of the corresponding second terminal (2) by means of protrusions (12, 13) in its lower border, while the other part (18) of the central body (5) receives in its cuplike end protrusions constituted on the end of the corresponding first terminal (1) of the catheter tube (3).

4. Disinfecting connection for catheters and the like according to claim 3, **characterized in that** the second part (15) of the central body (5) receiving the needle (11) has an intermediate partition (19) having a hole (20) for the guidance and passage of the needle (11), said partition (19) abutting one of the end faces of the disinfecting element (6), the disinfecting element (6) having a cylindrical body fitted within the first part (18) of the central body (5) and having end closures (22, 25) made of an elastic material penetratable by the needle (11) and limiting the chamber carrying a disinfecting liquid.

5. Disinfecting connection for catheters and the like according to claim 3, **characterized in that** the first and second parts (29, 30) of the central body (5) supporting the disinfecting element (6) are permanently assembled together, thereby preventing its disassembly.

6. Disinfecting connection for catheters and the like according to claim 5, **characterized in that** the first and second parts (29, 30) of the central body (5) are permanently assembled together by means of welding or similar of the abutment areas (31, 32) of said parts (29, 30) to a protruding ring (33) of the disinfecting element (6).

## Patentansprüche

1. Desinfizierende Kupplung für Katheter und dergleichen, die eine ein desinfizierendes Produkt enthaltende Kammer umfaßt, **dadurch gekennzeichnet**, daß die Kammer ein mehrfach verwendbares Desinfektionselement (6) ist, daß in einem entsprechenden Raum eines länglichen Zentralkörpers (5) enthalten ist, wobei der Körper einen ersten Teil (18) und einen zweiten Teil (15) umfaßt, die miteinander zusammenwirken, um den Raum zu bilden, wobei der Zentralkörper (5) in einem seiner Enden einen ersten Terminal (1) empfängt, der an einem ersten Katheterschlauch (3) angeschlossen ist, und an seinem anderen Ende ein zweites Terminal (2) empfängt, daß an einen zweiten Schlauch (4) angeschlossen ist, und eine Nadel (11) trägt, die dazu bestimmt ist, das kammerartige mehrfachverwendbare Desinfektionselement (6) zu durchqueren.

2. Desinfizierende Kupplung für Katheter und dergleichen gemäß Anspruch 1, **dadurch gekennzeichnet**, daß der die Nadel (11) tragende Zweitterminal (2) mit einem seiner Enden auf eine Vergrößerung (7) des zugehörigen zweiten Schlauchs (4) ausgepreßt ist, wahrend es in dem anderen Ende einen becherartigen Abschnitt (8) mit Innengewinde zur Aufnahme des zugehörigen Teils des das des Infektionselement (6) tragenden Zentralkörper (5) besitzt, wobei der zweite Terminal (2) desweiteren eine axiale Verlangerung (9) besitzt, die von dem becherartigen Abschnitt (8) hervorragt und den Preßsitz der Basis der Nadel (11) gestattet.

3. Desinfizierende Kupplung für Katheter und dergleichen gemäß Anspruch 1, **dadurch gekennzeichnet**, daß der zweite Abschnitt (15) des Zentralkörpers (5) mit dem Innengewinde des zugehörigen zweiten Terminals (2) mittels Vorsprüngen (12, 13) in dessen unterem Rand zusammenpaßt, während der andere Teil (18) des Zentralkörpers (5) in seinem becherartigem Ende Vorsprünge aufnimmt, die auf dem Ende des zugehörigen ersten Terminals (1) des Katheterschlauchs (3) ausgebildet sind.

4. Desinfizierende Kupplung für Katheter und dergleichen gemäß Anspruch 3, **dadurch gekennzeichnet**, daß der die Nadel (11) empfangende zweite Teil (15) des Zentralkörpers (5) eine Zwischentrennwand (19) mit einem Loch (20) zur Führung und zum Durchtritt der Nadel (11) besitzt, wobei die Trennwand (19) gegen eine der Endflächen des Desinfektionselements (6) anstößt, wobei das Desinfektionselement (6) einen zylindrischen Körper besitzt, der in dem ersten Teil (18) des Zentralkörpers (5) eingepaßt ist und Endverschlüsse (22, 25) besitzt, die aus einem elastischen, von der Nadel (11) durchdringbaren Material bestehen und die eine Desinfektionsflüssigkeit tragende Kammer begrenzen.

5. Desinfizierende Kupplung für Katheter und dergleichen gemäß Anspruch 3, **dadurch gekennzeichnet**, daß die ersten und zweiten Teile (29, 30) des Zentralkörpers (5), der das Desinfektionselement (6) trägt, permanent miteinander verbunden sind, wodurch ihr Auseinandernehmen verhindert wird.

6. Desinfizierende Kupplung für Katheter und dergleichen gemäß Anspruch 5, **dadurch gekennzeichnet**, daß die ersten und zweiten Teile (29, 30) des Zentralkörpers (5) permanent miteinander verbunden sind, in dem die Anstoßbereiche (31, 32) der Teile (29, 30) mit einem vorstehenden Ring (33) des Desinfektionselements (6) verschweißt oder auf ähnliche Weise verbunden werden.

## Revendications

1. Raccord désinfectant pour cathéters et analogues, qui comprend une chambre contenant un produit désinfectant, caractérisé en ce que ladite chambre est un élément désinfectant à usages multiples (6) contenu à l'intérieur d'un espace correspondant d'un corps central longitudinal (5), ledit corps comprenant une première partie (18) et une seconde partie (15) pouvant engrener réciproquement pour former ledit espace, ledit corps central (5) recevant, dans l'une de ses extrémités, un premier embout (1), qui est couplé à un premier tube de cathéter (3) et reçoit à son autre extrémité un second embout (2), qui est couplé à un second tube (4), et le second embout porte une aiguille (11) destinée à traverser ledit élément désinfectant à usages multiples en forme de chambre (6).

2. Raccord désinfectant pour cathéters et analogues selon la revendication 1, caractérisé en ce que le second embout (2) portant l'aiguille (11) est enfiché sous pression par l'une de ses extrémités sur un prolongement (7) du second tube correspondant (4), tandis que dans l'autre extrémité, il comporte une partie en forme de pot (8) possédant un taraudage servant à loger la partie correspondante du corps central (5) portant l'élément désinfectant (6), le second embout (2) possédant au moins un élargissement axial (9) qui fait saillie à partir de la partie en forme de pot (8) et permet un engrènement sous pression de la base de l'aiguille (11).

3. Raccord désinfectant pour cathéters et analogues selon la revendication 1, caractérisé en ce que la seconde partie (15) du corps central (5) engrène avec le taraudage du second embout correspondant (2) au moyen de parties saillantes (12,13) dans son bord inférieur, tandis que l'autre partie (18) du corps central (5) reçoit, dans son extrémité en forme de pot, les parties saillantes formées sur l'extrémité du premier embout correspondante (1) du tube (3) du cathéter.

4. Raccord désinfectant pour cathéters et analogues selon la revendication 3, caractérisé en ce que la seconde partie (15) du corps central (5) recevant l'aiguille (11) possède une cloison de séparation intermédiaire (19) possédant un trou (20) pour le guidage et le passage de l'aiguille (11), ladite cloison de séparation (19) étant en butée contre l'une des faces de l'élément désinfectant (6), l'élément désinfectant (6) possédant un corps cylindrique monté dans la première partie (18) du corps central (5) et possédant des dispositifs de fermeture d'extrémité (22,25) réalisés en un matériau élastique dans lequel peut pénétrer l'aiguille (11), et limitant la chambre contenant un liquide désinfectant.

5. Raccord désinfectant pour cathéters et analogues selon la revendication 3, caractérisé en ce que les première et seconde parties (29,30) du corps central (5) supportant l'élément désinfectant (6) sont assemblées de façon permanente, ce qui empêche leur démontage.

6. Raccord désinfectant pour cathéters et analogues selon la revendication 5, caractérisé en ce que les première et seconde parties (29,30) du corps central (5) sont assemblées de façon permanente par soudage ou analogue des zones de butée (31,32) desdites parties (29,30) sur un anneau saillant (33) de l'élément désinfectant (6).
